# FASCICULE DE BREVET EUROPEEN

(11) **EP 2 247 181 B1**
(45) Date de publication et mention de la délivrance du brevet: **23.11.2011**
(21) Numéro de dépôt: 08872688.0
(22) Date de dépôt: 24.12.2008
(51) Int. Cl.: A01N 1/02, C12N 5/02, A61K 35/52

(54) **MÉTHODE DE CONSERVATION DU SPERME ET SES APPLICATIONS.**
VERFAHREN ZUR SPERMIENKONSERVIERUNG UND SEINE ANWENDUNG
METHOD FOR PRESERVING SPERM AND APPLICATIONS THEREOF

(30) Priorité: 27.12.2007 FR 0709145
(43) Date de publication de la demande: 10.11.2010
(73) Titulaire: Institut National De La Recherche Agronomique, 75007 Paris (FR)
(72) Inventeur: MAGISTRINI, Michèle, F-37550 Saint-avertin (FR); PILLET, Elodie, F-73390 Chamousset (FR); BATELLIER, Florence, F-37000 Tours (FR); DUCHAMP, Guy, F-37360 Sonzay (FR)
(74) Mandataire: Métay, Émeline
(86) Numéro de dépôt international: PCT/FR2008/001822
(87) Numéro de publication internationale: WO 2009/103908

(56) Documents cités:
- WO-A-98/37904
- BATELLIER F ET AL: "Advances in cooled semen technology" ANIMAL REPRODUCTION SCIENCE, ELSEVIER SCIENCE PUBLISHERS, AMSTERDAM, NL, vol. 68, no. 3-4, 3 décembre 2001 (2001-12-03), pages 181-190, XP002385349 ISSN: 0378-4320 cité dans la demande
- VIDAMENT ET AL: "French field results (1985-2005) on factors affecting fertility of frozen stallion semen" ANIMAL REPRODUCTION SCIENCE, ELSEVIER SCIENCE PUBLISHERS, AMSTERDAM, NL, vol. 89, no. 1-4, 1 octobre 2005 (2005-10-01), pages 115-136, XP005084218 ISSN: 0378-4320 cité dans la demande
- LEBOEUF B ET AL: "Effect of native phosphocaseinate on the in vitro preservation of fresh semen" THERIOGENOLOGY, LOS ALTOS, CA, US, vol. 60, no. 5, 15 septembre 2003 (2003-09-15), pages 867-877, XP009098941 ISSN: 0093-691X
- BERGERON A ET AL: "New insights towards understanding the mechanisms of sperm protection by egg yolk and milk" MOLECULAR REPRODUCTION AND DEVELOPMENT 200610 US, vol. 73, no. 10, octobre 2006 (2006-10), pages 1338-1344, XP002482512 ISSN: 1040-452X 1098-2795
- VIDAMENT MARIANNE ET AL: "Differences in ability of jennies and mares to conceive with cooled and frozen semen containing glycerol or not." ANIMAL REPRODUCTION SCIENCE MAY 2009, vol. 112, no. 1-2, mai 2009 (2009-05), pages 22-35, XP002538217 ISSN: 1873-2232
- ROTA ALESSANDRA ET AL: "Effect of extender, centrifugation and removal of seminal plasma on cooled-preserved Amiata donkey spermatozoa." THERIOGENOLOGY 15 JAN 2008, vol. 69, no. 2, 15 janvier 2008 (2008-01-15), pages 176-185, XP022400695 ISSN: 0093-691X

## Description

La présente Invention concerne le domaine de la conservation du sperme, et plus particulièrement la préservation de l'aptitude à la fécondation des spermatozoïdes fragilisés par la congélation ou un autre traitement.

Avec le développement des biotechnologies de la reproduction, les techniques d'insémination artificielle sont pratiquées couramment chez de nombreuses espèces animales, notamment caprine, ovine, porcine, bovine et équine. Cependant, les spermatozoïdes sont des cellules complexes hautement spécialisées qui peuvent perdre rapidement leur pouvoir fécondant. Ainsi, la conservation de longue durée à l'état frais (températures positives) ou à l'état congelé du sperme tout en maintenant son pouvoir fécondant pose un problème.

Aussi afin de permettre une conservation optimale du sperme jusqu'à son utilisation pour l'insémination artificielle, on utilise habituellement un milieu dans lequel on dilue le sperme aussitôt après sa récolte. Le mélange sperme/milieu de dilution est alors réparti en aliquotes contenant un nombre de spermatozoïdes suffisant pour féconder l'ovule, par exemple au minimum 2.10⁸ spermatozoïdes chez les chevaux lors d'inséminations artificielles de semence « fraîche ». Selon le milieu de dilution utilisé et la température à laquelle le mélange sperme/milieu est conservé, la durée de conservation varie en général de quelques heures à plusieurs jours, voire une période plus longue dans les cas où le milieu permet la congélation. Le sperme conservé entre 4 et 20°C a le désavantage de devoir être utilisé dans les heures ou les 2-3 jours suivant la collecte. *A contrario,* la conservation du sperme par congélation permet de conserver la semence d'un mâle pendant plusieurs années. La congélation du sperme permet également de conserver le patrimoine génétique d'un mâle de haute valeur génétique et de le valoriser éventuellement ensuite après sa mort ou sa castration. La congélation du sperme permet en outre le transport de semences vers des élevages géographiquement éloignés du lieu de la collecte, ce qui facilite l'échange et la diffusion de la génétique ou la vente internationale de semence.

Cependant, il a été démontré que le processus de congélation/décongélation diminuait le pourcentage de spermatozoïdes intacts (Watson. P.F., Anim. Reprod. Sci. 60-61 :481-492, 2000) : la congélation présente l'inconvénient de provoquer la dégradation de fonctions physiologiques importantes des spermatozoïdes diminuant partiellement ou totalement leur pouvoir fécondant. Puisque la qualité de la semence est primordiale lors de l'insémination artificielle, une dose d'insémination contenant un nombre important de spermatozoïdes morts ou fragilisés aura un pouvoir fécondant diminué, limitant ainsi la fertilité du mâle après insémination.

La mise au point d'un milieu de dilution permettant d'améliorer la conservation de la semence et de préserver une bonne capacité fécondante des spermatozoïdes lors de leur congélation fait donc l'objet de nombreuses recherches.

Chez l'étalon, le milieu qui a permis les premières fécondations après insémination artificielle de semence congelée était constitué de lait entier pasteurisé contenant 10% de glycérol (Barker et al. Canadian Journal of Comparative Medical Veterinary Science, 21 :47-51, 1957)

Parmi les milieux de dilution les plus fréquemment utilisés pour la congélation de la semence d'étalon, on citera en particulier les milieux décrits par Martin et al (J. Reprod. Fertil., Suppl. 27 :47-51, 1979), Burns P.J. (Proc. 12th International Congress on Animal Reproduction The Hague 1992 ; 4 :1849-1851), et le milieu dénommé "INRA82", décrit par Palmer E. (Proc. 10th International Congress on Animal Reproduction and Artificial Insemination, Urbana-Champaign, IL USA, 3:377, 1984), modifié par Magistrini et al. (Acta Veterinaria Scandinavica, 1st European Symposium on Production, Evaluation and Preservation of Stallion Semen, 1-2 october 1992, Uppsala, Sweden, Suppl. 88, 97-110) et constitué d'un milieu de base additionné de lait écrémé UHT.

Généralement, ces milieux de congélation sont supplémentés avec diverses substances, dans le but d'améliorer la protection des spermatozoïdes. Vidament et al. (Theriogenology., 54, 907-19, 2000) ont fait évoluer la technique décrite par Palmer E. (1984) : le sperme est dilué dans le milieu INRA82 supplémenté avec du jaune d'oeuf et centrifugé, puis le culot de centrifugation est repris dans le même milieu supplémenté en outre avec du glycérol, préalablement à la congélation. Les meilleurs résultats sont obtenus en effectuant la centrifugation et l'addition de glycérol à une température de 22°C, puis un refroidissement graduel jusqu'à 4°C préalablement à la mise en paillettes et à la congélation ; dans ces conditions, on observe non seulement une amélioration de la mobilité des spermatozoïdes après décongélation, mais aussi une amélioration de la fertilité.

Cependant, même dans ces conditions optimisées, le pouvoir fécondant des spermatozoïdes après décongélation demeure inférieur à celui de la semence fraîche. Une étude menée en France sur la période 1985-2005 dans les Haras Nationaux français rapporte que le taux de gestation obtenu après insémination artificielle avec de la semence d'étalon congelée dans le milieu INRA82 précité supplémenté de jaune d'oeuf et de glycérol dans les conditions indiquées précédemment, et utilisant de la semence dont la mobilité après décongélation est supérieure à 35%, était de 45-48% (Vidament. M., Anim. Reprod. Sci. 89 :115-136, 2005) comparé à 55% pour la semence fraîche.

A côté des milieux de dilution du sperme destinés à la congélation, il en existe également d'autres destinés à la conservation des spermatozoïdes à des températures positives. Ainsi, la Demande PCT WO9837904 décrit un milieu de dilution du sperme constitué d'un milieu de base comprenant, en remplacement du lait, du phosphocaséinate natif, et/ou de la β-lactoglobuline. A une température de 4°C, le milieu de dilution contenant du phosphocaséinate natif présente, in vitro, des performances similaires, voire parfois inférieures, à celles du milieu INRA82 constitué d'un milieu de base additionné de lait écrémé UHT; en revanche, il améliore très significativement la conservation du sperme à 15°C.

Les Inventeurs ont testé les effets d'un milieu de dilution du sperme, tel que décrit dans la Demande PCT WO9837904, supplémenté avec du jaune d'oeuf et du glycérol, sur la conservation des spermatozoïdes lors de la congélation. Des premiers essais ont montré que ce milieu n'améliorait pas les paramètres de mobilité des spermatozoïdes après décongélation, par rapport à un milieu de base supplémenté de lait (l'INRA82 précité), et de jaune d'oeuf et de glycérol. En revanche, les Inventeurs ont maintenant constaté que, de manière surprenante, il améliorait considérablement le pouvoir fécondant des spermatozoïdes.

Cette amélioration du pouvoir fécondant apparait liée à la capacité de ce milieu à préserver efficacement l'intégrité de la membrane des spermatozoïdes lors du stress résultant de la congélation/décongélation. Il peut donc être utilisé pour améliorer le pouvoir fécondant du sperme fragilisé, non seulement par la congélation, mais par toute autre manipulation susceptible d'avoir un effet délétère sur les membranes des spermatozoïdes. Il s'agit notamment de la cytométrie de flux, utilisée chez certaines espèces d'animaux d'élevage, pour séparer les spermatozoïdes Y des spermatozoïdes X préalablement à l'insémination.

La présente Invention a pour objet l'utilisation d'un milieu de dilution du sperme, constitué par :
- un milieu de base comprenant les constituants convenant pour la dilution du sperme d'une espèce déterminée ;
- du phosphocaséinate natif ;
- du jaune d'oeuf ou du plasma de jaune d'oeuf ;
   et
- du glycérol ;
   pour améliorer l'aptitude à la fécondation des spermatozoïdes fragilisés ou endommagés par la congélation et/ou par le tri des spermatozoïdes en cytométrie de flux.

Le milieu de base selon la présente Invention inclut une solution de sels minéraux et de glucides, et n'inclut pas de lait. Le jaune d'oeuf est constitué d'un fluide dans lequel sont suspendues diverses particules (ANTON, Recent Res. Devel. in Agricultural & Food Chem., 2, 839-864, 1998). C'est ce fluide qui est dénommé : « plasma de jaune d'oeuf ». Ces deux phases peuvent être facilement séparées par centrifugation. Cette séparation est généralement effectuée en diluant le jaune d'oeuf (pour diminuer sa viscosité), dans de l'eau ou une solution faiblement saline (typiquement une solution de NaCl de molarité inférieure à 0,3 M, de préférence inférieure à 0,2 M), et en soumettant le mélange ainsi obtenu à une centrifugation dans des conditions (par exemple 10000 g pendant 30 minutes) permettant de séparer le plasma, qui constitue le surnageant, de la fraction « granules », qui sédimente dans le culot. Avantageusement, on peut, si on le souhaite, effectuer une seconde centrifugation, afin d'éliminer plus complètement la fraction granules.

Généralement, le plasma de jaune d'oeuf sera en outre stérilisé, par exemple par irradiation gamma.

Par « un milieu de base comprenant les constituants convenant pour la dilution du sperme d'une espèce déterminée », on entend tout milieu comprenant les constituants définis chimiquement, habituellement utilisés dans les milieux de dilution du sperme utilisés pour ladite espèce. Il s'agit généralement d'une solution de sels minéraux et de glucides, à un pH approprié. La nature et les proportions de ces différents constituants peuvent varier selon l'espèce concernée. Ce milieu de base peut comprendre également des additifs tels que des antibiotiques ou des antifongiques.

Selon la présente Invention, le milieu de dilution du sperme utilisé comprend :
- de 1 à 10%, et de préférence de 1,5 à 5% de glycérol ;
- de 0,5 à 12,5%, de préférence de 0,5 à 5%, et de manière tout à fait préférée, de 1 à 2,5% en poids de matière sèche, de jaune d'oeuf, ou bien de 0,4 à 10%, de préférence de 0,4 à 4%, et de manière tout à fait préférée de 0,8 à 2% en poids de matière sèche de plasma de jaune d'oeuf ;
- entre 1 et 100 g/l, de préférence entre 10 et 50 g/l de phosphocaséinate natif.

Le jaune d'oeuf est constitué d'environ 50% de matière sèche, et le plasma de jaune d'oeuf représente environ 80% de la matière sèche du jaune d'oeuf : en conséquence, 2% de jaune d'oeuf frais apportent environ 1% en poids de matière sèche de jaune d'oeuf, et environ 0,8% en poids de matière sèche de plasma de jaune d'oeuf.

La présente invention peut être mise en oeuvre dans le cadre de l'insémination artificielle chez des mammifères de différentes espèces, notamment chez les espèces caprine, ovine, porcine, bovine et équine, et de manière particulièrement avantageuse chez les espèces bovine et équine, selon les modalités usuelles d'insémination artificielle pour l'espèce concernée, qui sont connues en elles-mêmes de l'homme du métier.

La présente Invention sera mieux comprise à l'aide du complément de description qui va suivre, qui se réfère à des exemples non-limitatifs de son utilisation chez le cheval.

### EXEMPLE 1 - MATERIELS ET METHODES

### a) Milieux de dilution du sperme

Les deux milieux de dilution du sperme utilisés dans les tests comparatifs présentés ci-après sont les milieux « INRA82 » et « INRA96® ». Le milieu « INRA82 » est un mélange de 0,5 litre d'un milieu de base (solution saline-glucose : glucose 25 g.L⁻¹, lactose 1,5 g.L⁻¹, raffinose 1,5 g.L⁻¹, citrate de sodium déshydraté 0,25 g.L⁻¹, citrate de potassium 0,41 g.L⁻¹, hépès 4,76 g.L⁻¹) avec 0,5 litre de lait, à pH 6,8. Le milieu « INRA96® » est décrit dans la Demande PCT WO9837904, ainsi que dans la publication de Batellier et al., 1997 Theriogenology, 48-3, 391-410) : il est constitué d'un milieu de base (milieu HGLL, constitué de sels de Hank's supplémentés de tampon hépès, de lactose et de glucose) et de 27g/L de phosphocaséinate natif.

Les deux milieux contiennent également 50 000 UI.L⁻¹ de pénicilline et 50 mg.L⁻¹ de gentamicine.

Les milieux INRA82 et Infra96® ont également été supplémentés avec 2% de jaune d'oeuf centrifugé (à 600 x g pendant 10 min. pour éliminer les éventuelles contaminations par du blanc, de la chalaze, ou des débris de coquille) et 2,5% de glycérol : ces milieux seront respectivement nommés INRA82+JO+G et INRA96®+JO+G par la suite.

A titre de comparaison, le milieu INFRA96® supplémenté uniquement avec 2,5% de glycérol a également été testé. Ce milieu est nommé ci-après INRA96®+G.

### b) Collecte du sperme et préparation

Trois étalons Welsh adultes, de fertilité connue, ont été utilisés pour cette étude. Ils sont hébergés dans l'unité expérimentale de l'Institut National de la Recherche Agronomique (INRA) de Nouzilly, France. Sept éjaculats par étalon ont été traités. Après la récolte, la semence a été filtrée sur de la gaze afin d'enlever la fraction « gel » provenant des vésicules séminales. Immédiatement après la filtration, chaque éjaculat a été divisé en deux aliquotes qui ont été dilués dans les milieux INRA82 ou Infra96®, la dilution minimale étant de 1 volume de semence pour 3 de milieu. Le mélange a ensuite été refroidi à 22°C durant 10 minutes puis centrifugé à 600g durant 10 minutes. Le culot a alors été remis en suspension, soit dans le milieu INRA82+JO+G, soit dans le milieu INRA96®+JO+G, selon le type de milieu utilisé pour la première étape, afin d'obtenir une concentration finale de 100.10⁶ spermatozoïdes par ml. Chaque échantillon a été refroidi à 4°C pendant 75 minutes, mis en paillettes de congélation de 0.5mL en chlorure de polyvinyle scellées par une poudre polymérisante. La congélation a été réalisée à l'aide d'un congélateur programmable pour abaisser la température de 4°C à -140°C à la vitesse de 60°C par minute. Les paillettes sont alors conservées dans de l'azote liquide à -196°C, puis décongelées dans un bain-marie pendant 30 secondes à 37°C immédiatement avant les analyses ou les inséminations artificielles.

### EXEMPLE 2 - EVALUATION IN VITRO DE LA QUALITE DU SPERME APRES CONGELATION

La mobilité des spermatozoïdes et la résistance de leur membrane plasmique à une gamme de pressions hypo-osmotiques ont été analysées par analyse automatisée de la mobilité assistée par ordinateur ou par cytométrie en flux respectivement.

### a) Mobilité des spermatozoïdes

Pour évaluer la mobilité des spermatozoïdes, trois paillettes de chacun des 7 éjaculats et pour chacun des 3 étalons ont été décongelées puis diluées à une concentration de 20.10⁶ spermatozoïdes par ml dans chacun des milieux INRA82 ou INRA96®. Avant l'analyse, les échantillons de sperme dilué sont incubés 10 minutes à 37°C. Les trois paramètres suivants de mobilité des spermatozoïdes sont évalués par un analyseur automatisé de mobilité (IVOS, Version 10, Hamilton Thorne, Beverly, MA, USA) : la vitesse moyenne. (VAP) en µm.s⁻¹, le pourcentage de spermatozoïdes progressifs (PROG) et le pourcentage de spermatozoïdes rapides (RAP : spermatozoïdes ayant une vitesse moyenne supérieure à 30 µm. s⁻¹). Les analyses ont été réalisées sur 2 gouttes par échantillons et par paillette et 3 champs optiques par goutte.

### Comparaison entre le milieu INRA82+JO+G, et le milieu INRA96®+JO+G.

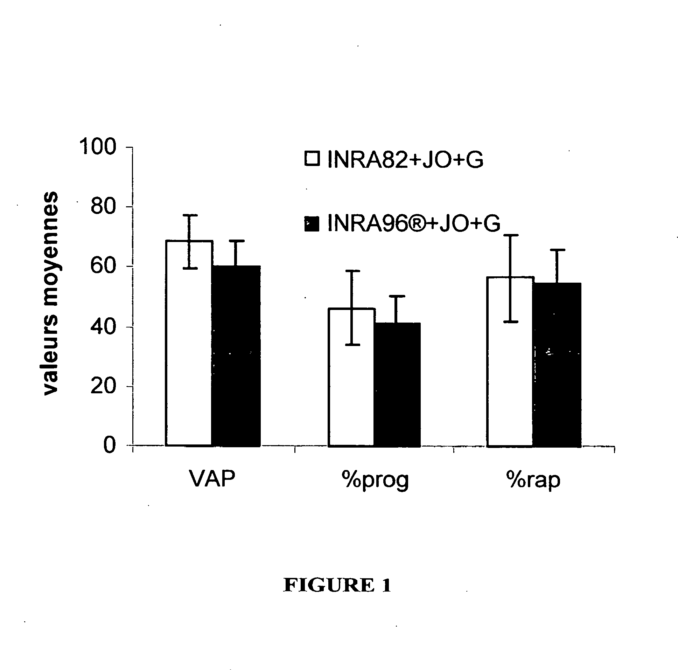

La Figure 1 est un histogramme montrant les résultats obtenus pour les paramètres de mobilité VAP, PROG et RAP. Les barres blanches représentent les résultats de chacun des trois paramètres évalués après congélation des spermatozoïdes dans le milieu INRA82+JO+G, les barres gris foncé représentent les résultats de chacun des trois paramètres évalués après congélation des spermatozoïdes dans le milieu INRA96®+JO+G. Les barres d'erreur correspondent à la déviation standard.

Ces résultats montrent que la vitesse moyenne, le pourcentage de spermatozoïdes progressifs et le pourcentage de spermatozoïdes rapides sont significativement supérieurs lorsque la semence est congelée dans le milieu INRA82+JO+G comparé au milieu INRA96®+JO+G (VAP : 82 µm.s⁻¹ *versus* 66 µm. s⁻¹, p<0,0001; PROG : 51% *versus* 45%, p<0,0001, RAP : 61% *versus* 58%, p<0,01). Il ressort de cette analyse que lors de la congélation dans ces conditions expérimentales, le milieu INRA82+JO+G préserve mieux la mobilité des spermatozoïdes que le milieu INRA96®+JO+G.

### Comparaison entre le milieu INRA96+G, et le milieu INRA96®+JO+G.

Pour comparer les milieux INRA96+G, et INRA96®+JO+G, 10 éjaculats provenant de 5 étalons, à raison de 2 éjaculats par étalons, ont été collectés, congelés, et décongelés, dans les mêmes conditions que celles décrites ci-dessus.

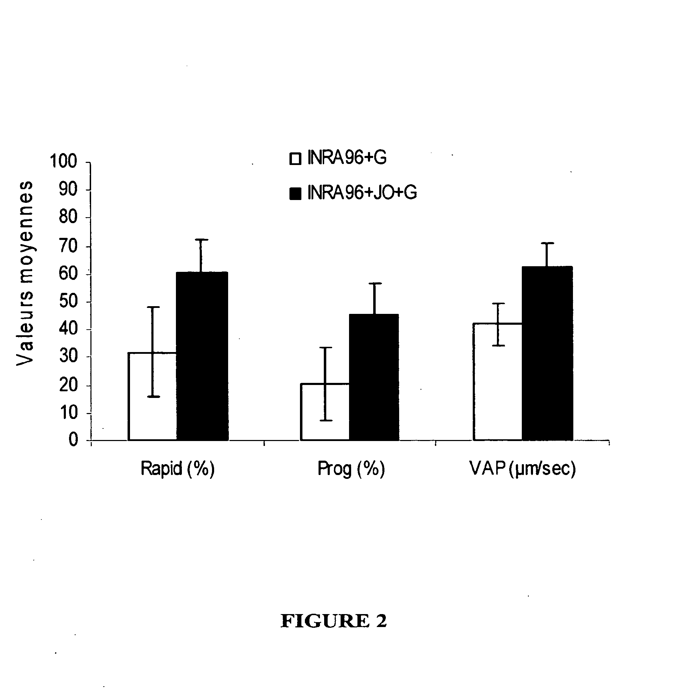

La Figure 2 est un histogramme présentant les résultats obtenus pour les paramètres de mobilité VAP, PROG et RAP. Les barres blanches représentent les résultats des paramètres évalués après congélation des spermatozoïdes dans le milieu INRA96+G, les barres noires représentent les résultats des paramètres évalués après congélation des spermatozoïdes dans le milieu INRA96®+JO+G. Les barres d'erreur correspondent à la déviation standard.

Ces résultats montrent clairement que la vitesse moyenne (VAP), le pourcentage de spermatozoïdes progressifs (PROG) et le pourcentage de spermatozoïdes rapides (RAPID) sont significativement supérieurs lorsque la semence est congelée dans le milieu INRA96®+JO+G par rapport au milieu INRA96®+G (VAP : 63 µm.s⁻¹ *versus* 42 µm.s⁻¹, p<0.001; PROG : 45,5% *versus* 20%, p<0.001, RAP : 60% *versus* 32%, p<0.001). Ces résultats montrent donc que lors de la congélation, le milieu INRA96 nécessite d'être complémenté conjointement avec du glycérol et du jaune d'oeuf afin de préserver au mieux la mobilité des spermatozoïdes.

### b) Intégrité de la membrane plasmique

L'évaluation de l'intégrité des membranes des spermatozoïdes a été réalisée selon la méthode décrite dans l'espèce caprine par Leboeuf et al. (Animal Reproduction Science, 91,3-4 : 265-274, 2006) et dans l'espèce équine par Defoin et al. (Anim. Reprod. Sci. 89 :219-223, 2005). Ceci consiste à tester la résistance de la membrane des spermatozoïdes soumis à un stress hypo-osmotique. La semence est décongelée (deux paillettes par éjaculat et cinq éjaculats par étalon) et diluée dans chacun des milieux INRA82 ou Infra96® à la concentration de 20.10⁶ spermatozoïdes par ml. Les spermatozoïdes dilués ont été immédiatement centrifugés à 500g pendant 4 minutes, puis le culot remis en suspension dans des solutions salines de Hank's (Hank's salts solution), supplémentées avec de l'hépes 20mM, de pression osmotique décroissante (303, 205, 154, 103, 63, 35, 12 mOsm), est ensuite incubé 15 minutes à 37°C. Les spermatozoïdes sont alors dilués à 1.10⁶ spermatozoïdes par ml, marqués à l'iodure de propidium (IP, concentration finale 2,5 µg.mL⁻¹), puis incubés à nouveau 5 minutes à 37°C avant l'analyse en cytométrie en flux (moFlo® cell sorter, Darko society, Danemark) de l'incorporation de l'IP par les spermatozoïdes.

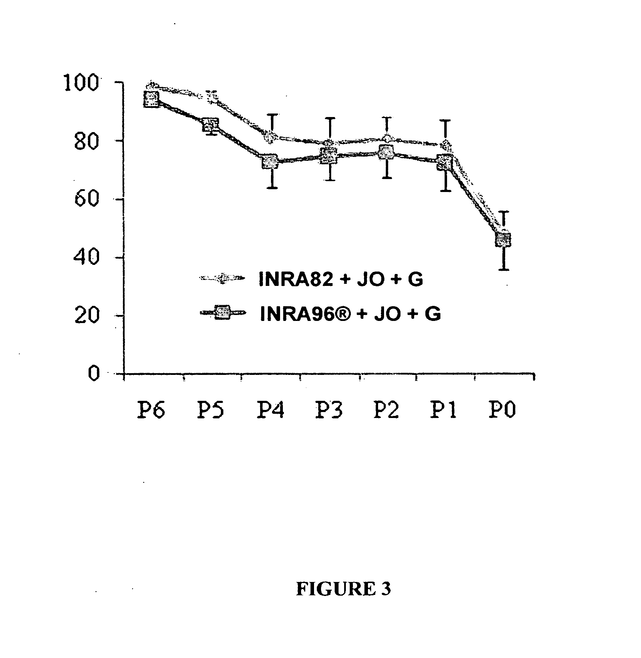

La Figure 3 est un diagramme qui représente les résultats obtenus lors des tests de résistance de la membrane des spermatozoïdes. Les pressions testées sont indiquées en abscisse : P0, P1, P2, P3, P4, P5 et P6 correspondent respectivement aux pressions osmotiques 303, 205, 154, 103, 63, 35, 12 mOsm. Le pourcentage de spermatozoïdes ayant incorporé l'IP est indiqué en ordonnée. La courbe blanche présente les résultats obtenus après congélation dans le milieu INRA82+JO+G, la courbe grisée ceux obtenus après congélation dans le milieu INRA96®+JO+G. Les barres d'erreur correspondent à la déviation standard.
La Figure 3 montre que lors d'un stress osmotique hypotonique, quelle que soit la pression osmotique testée (205, 154, 103, 63, 35, 12 mOsm), le pourcentage de spermatozoïdes ayant incorporé l'IP, qui correspond au pourcentage de spermatozoïdes présentant une membrane endommagée, est significativement inférieur (p<0,05) lorsque la semence est congelée dans le milieu INRA96®+JO+G comparée au milieu INRA82+JO+G. En revanche, dans le milieu à 303 mOsm (iso-osmotique pour les spermatozoïdes), l'incorporation de l'IP est identique entre les spermatozoïdes congelés dans le milieu INRA82+JO+G et ceux congelés dans le milieu INRA96®+JO+G. En outre, quel que soit le milieu utilisé, le pourcentage de spermatozoïdes ayant incorporé l'IP (donc le pourcentage de spermatozoïdes présentant une membrane endommagée) augmente fortement dans le milieu hypo-osmotique à 205 mOsm par rapport au milieu à 303 mOsm. Ces résultats indiquent donc que le milieu INRA96®+JO+G préserve mieux l'intégrité des membranes des spermatozoïdes lors de la congélation.

### EXEMPLE 3 - EVALUATION IN VIVO DU POUVOIR FECONDANT DU SPERME APRES CONGELATION

Les ponettes ont été échographiées chaque jour à partir du premier jour des chaleurs jusqu'à l'ovulation. Lorsque qu'un follicule dominant a atteint 35 mm, l'ovulation a été induite par injection intraveineuse de 15 mg de C.E.G (crude equine pituitary gonadotrophin ; Duchamp et al., Journal of Reproduction and Fertility, 35 : 221-228, 1987) (Jour J0), puis les ponettes ont été inséminées le jour suivant (J1) avec 400.10⁶ spermatozoïdes précédemment congelés dans l'un ou l'autre des milieux INRA96®+JO+G ou INRA82+JO+G (volume inséminé : 4 ml, soit 8 paillettes décongelées et regroupées. La répartition des ponettes entre les deux lots a été faite au hasard. Les 7 éjaculats utilisés lors des inséminations artificielles présentaient plus de 35% de spermatozoïdes rapides à la décongélation. Cette valeur est le minimum requis pour qu'un éjaculat soit utilisé en insémination artificielle dans les Haras Nationaux français.

Le diagnostic de gestation a été réalisé par échographie au 14^{ème} jour post-ovulation : le taux de fertilité par cycle a été calculé comme étant le nombre de cycles conduisant à une gestation par rapport au nombre total de cycles inséminés.

Quatre-vingt quatre cycles de ponettes ont été utilisés au total : 42 cycles avec des spermatozoïdes précédemment congelés dans le milieu INRA96®+JO+G, et 42 cycles avec des spermatozoïdes précédemment congelés dans le milieu INRA82+JO+G.

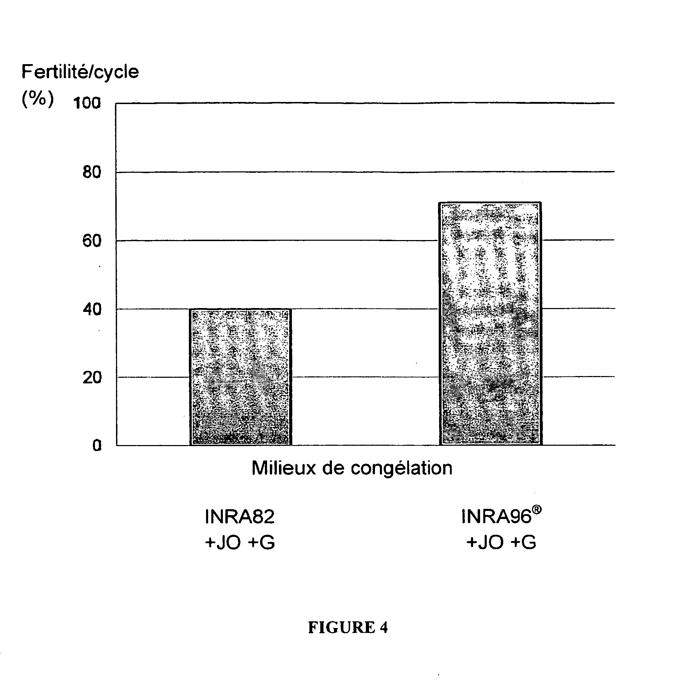

Les résultats sont présentés dans l'histogramme de la Figure 4. La fertilité par cycle est représentée en ordonnée, et les milieux de congélation INRA82+JO+G et INRA96®+JO+G sont indiqués en abscisse.

Ces résultats montrent que la fertilité par cycle obtenue après insémination artificielle avec des spermatozoïdes congelés dans le milieu INRA96®+JO+G est de 71% (30 gestations pour 42 inséminations), alors que celle obtenue avec des spermatozoïdes congelés dans le milieu INRA82+JO+G n'est que de 40% (17 gestations pour 42 inséminations).

En conclusion, le milieu INRA96® supplémenté de 2% de jaune d'oeuf centrifugé et de 2,5% de glycérol améliore significativement (p<0,01) l'aptitude à la fécondation de spermatozoïdes congelés par comparaison avec le milieu témoin INRA82+JO+G.

### EXEMPLE 4 - UTILISATION DU PLASMA DE JAUNE D'OEUF DANS LE MILIEU INRA96+G EN REMPLACEMENT DU JAUNE D'OEUF ENTIER

Le plasma de jaune d'oeuf a été préparé par dilution volume à volume de jaune d'oeuf entier dans du NaCl 0,17M, suivi d'une centrifugation à 10000 x g pendant 45 minutes. Le surnageant de cette centrifugation a été récupéré, et à nouveau centrifugé à 10000 x g pendant 45 minutes. Le surnageant de cette 2^{ème} centrifugation constitue le plasma de jaune d'oeuf qui a été utilisé pour la suite des expérimentations. Avant utilisation, ce plasma a été stérilisé par irradiation aux rayons gamma (à 5kGy). Ce plasma a été utilisé à raison de 4% en volume (soit environ 1% de matière sèche), pour supplémenter le milieu de dilution INRA96®.

Le milieu Infra96® supplémenté avec 4% de plasma de jaune d'oeuf et 2,5% de glycérol est nommé INRA96®+plasmaJO+G,

Des tests comparatifs ont été réalisés avec les milieux INRA96®+plasmaJO+G et INRA96®+JO+G.

Les protocoles de collecte et de préparation des échantillons de semence sont identiques à ceux décrits dans l'Exemple 1 ci-dessus.

### a) Evaluation in vitro de la qualité du sperme après congélation

L'évaluation des paramètres de mobilité des spermatozoïdes VAP, PROG et RAP a été réalisée par analyse automatisée comme décrit dans l'exemple 2.

* Une première étude *in vitro* a été réalisée avec la semence de six étalons Welsh adultes, à raison de deux éjaculats par étalon.

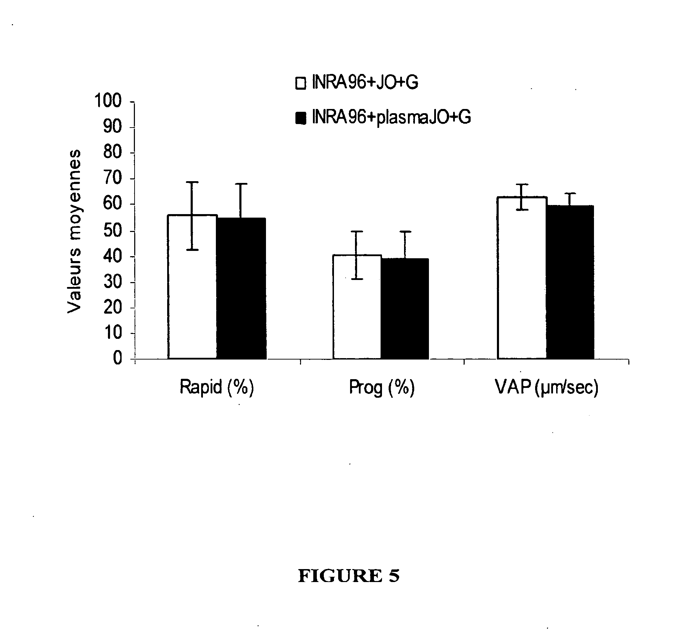

La Figure 5 est un histogramme montrant les résultats obtenus pour les paramètres de mobilité VAP, PROG et RAP. Les barres blanches représentent les résultats de chacun des trois paramètres évalués après congélation des spermatozoïdes dans le milieu INRA96®+JO+G, les barres noires représentent les résultats de chacun des trois paramètres évalués après congélation des spermatozoïdes dans le milieu INRA96®+plasmaJO+G. Les barres d'erreur correspondent à la déviation standard.

Ces résultats montrent que la vitesse moyenne (VAP) est significativement plus élevée lorsque la semence est congelée dans le milieu INRA96+JO+G comparé au milieu INRA96+plasmaJO+G (58µmsec⁻¹ vs 55.5µmsec⁻¹; p<0.05) ; en revanche, aucune différence n'a été mise en évidence entre les 2 milieux pour les paramètres pourcentage de spermatozoïdes rapides (Rapid) et pourcentage de spermatozoïdes progressifs (Prog) (44% vs 44% et 31% vs 30%, respectivement ; p>0.05).

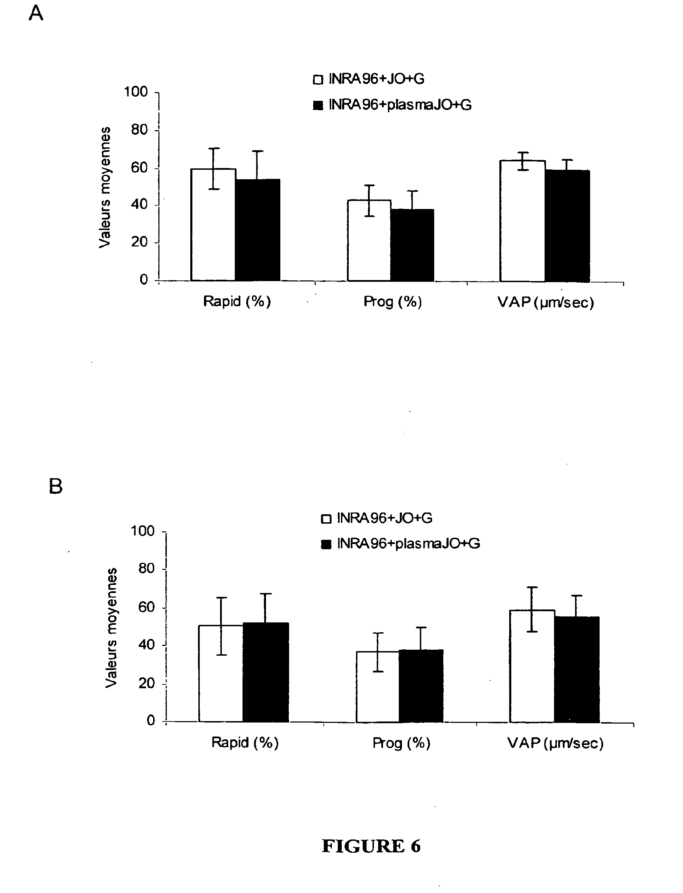

* Une seconde étude *in vitro* a été réalisée sur les éjaculats de deux étalons qui ont ensuite été utilisés pour un test de fertilité *in vivo* (cf. ci-après), à raison de quatre éjaculats pour l'étalon MW438 et cinq éjaculats pour l'étalon MW329. Les résultats de l'analyse automatisée obtenus pour l'étalon MW438 sont présentés sur la Figure 6.A, ceux obtenus pour l'étalon MW329 sont présentés sur la Figure 6.B.

Ces résultats montrent une légère augmentation de la vitesse moyenne des spermatozoïdes après congélation dans le milieu INRA96+JO+G. Il n'y a en revanche aucune différence entre les 2 milieux pour les paramètres « pourcentage de spermatozoïdes rapides » et « pourcentage de spermatozoïdes progressifs ». Ces résultats sont donc comparables aux résultats obtenus dans la première étude.

### B) Evaluation in vivo du pouvoir fécondant du sperme après congélation

L'évaluation de l'aptitude à la fécondation de spermatozoïdes des étalons MW438 et MW329, après congélation dans les milieux INRA96+plasmaJO+G ou INRA96+JO+G a été réalisée comme décrit dans l'exemple 3.

Soixante-dix cycles de ponettes ont été utilisés au total dans cette étude : 35 cycles avec des spermatozoïdes précédemment congelés dans le milieu INRA96®+plasmaJO+G, et 35 cycles avec des spermatozoïdes précédemment congelés dans le milieu INRA96®+JO+G. La fertilité de la semence des étalons MW438 et MW329 a été déterminée respectivement sur 19 et 16 cycles, pour chacun des milieux INRA96®+plasmaJO+G et INRA96®+JO+G.

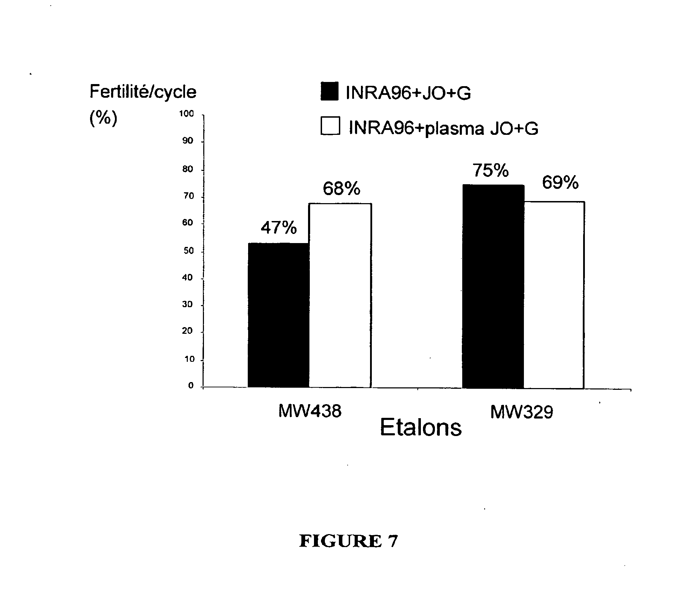

Les résultats sont présentés dans l'histogramme de la Figure 7. La fertilité par cycle est représentée en ordonnée, et les étalons sont indiqués en abscisse. Les barres blanches représentent les résultats obtenus après congélation de la semence dans le milieu INRA96®+plasmaJO+G, les barres noires représentent les résultats obtenus après congélation de la semence dans le milieu INRA96®+JO+G. Les barres d'erreur correspondent à la déviation standard.

Ces résultats montrent que la fertilité par cycle obtenue après insémination artificielle avec des spermatozoïdes provenant de l'étalon MW438 et de l'étalon MW329 congelés dans le milieu INRA96®+plasmaJO+G est respectivement de 68% et 69% (13 gestations pour 19 inséminations pour MW438 et 11 gestations pour 16 inséminations pour MW329). La fertilité par cycle après congélation dans le milieu INRA96®+JO+G est de 47% (9 gestations pour 19 inséminations) dans le cas de l'étalon MW438 et de 75% (12 gestations pour 16 inséminations) pour l'étalon MW329.

Ces résultats montrent que les spermatozoïdes congelés dans le milieu INRA96®+plasmaJO+G conservent un haut niveau de fertilité après congélation, comparable à celui observé après congélation avec le milieu INRA96®+JO+G.

En conclusion, ces résultats *in vitro* et *in vivo* montrent que le jaune d'oeuf entier peut être remplacé dans le milieu selon l'invention par du plasma de jaune d'oeuf irradié sans affecter la qualité des spermatozoïdes, et en particulier leur aptitude à la fécondation.

## Revendications

1. Utilisation d'un milieu de dilution du sperme, constitué par :
- un milieu de base comprenant les constituants convenant pour la dilution du sperme d'une espèce déterminée, ledit milieu de base incluant une solution de sels minéraux et de glucides, et n'incluant pas de lait ;
- entre 1 et 100 g/l de phosphocaséinate natif;
- de 0,5 à 12,5% en poids de matière sèche de jaune d'oeuf ou de 0,4 à 10% en poids de matière sèche de plasma de jaune d'oeuf ; et
- de 1 à 10% de glycérol ;
pour améliorer l'aptitude à la fécondation du sperme fragilisé par la congélation ou par le tri des spermatozoïdes en cytométrie de flux.

2. Utilisation selon la revendication 1, **caractérisée en ce que** le milieu de dilution du sperme comprend entre 10 et 50 g/l de phosphocaséinate natif.

3. Utilisation selon la revendication 1 ou 2, **caractérisée en ce que** le milieu de dilution du sperme comprend de 0,5 à 5% en poids de matière sèche de jaune d'oeuf ou de 0,4 à 4% en poids de matière sèche de plasma de jaune d'oeuf.

4. Utilisation selon la revendication 3, **caractérisée en ce que** le milieu de dilution du sperme comprend de 1 à 2,5% de jaune d'oeuf ou de 0,8 à 2% en poids de matière sèche de plasma de jaune d'oeuf, et de 2 à 5% de glycérol.

5. Utilisation selon l'une quelconque des revendications 1 à 4, **caractérisée en ce que** le milieu de base comprend des additifs tels que des antibiotiques ou des antifongiques.

## Claims

1. Use of a medium for diluting sperm made up of:
- a base medium containing the constituents suitable for diluting the sperm of a given species, said base medium comprising a solution of mineral salts and glucides and not containing milk;
- between 1 and 100 g/l of native phosphocaseinate;
- 0.5 to 12.5 % by weight of egg yolk dry matter or 0.4 to 10 % by weight of egg yolk plasma dry matter; and
- 1 to 10 % of glycerol;
with a view to improving the fertilising capability of sperm weakened by freezing or by sorting the spermatozoa by flow cytometry.

2. Use as claimed in claim 1, **characterised in that** the sperm dilution medium comprises between 10 and 50 g/l native phosphocaseinate.

3. Use as claimed in claim 1 or 2, **characterised in that** the sperm dilution medium comprises 0.5 to 5 % by weight of egg yolk dry matter or 0.4 to 4 % by weight of egg yolk plasma dry matter.

4. Use as claimed in claim 3, **characterised in that** the sperm dilution medium comprises 1 to 2.5 % egg yolk or 0.8 to 2 % by weight of egg yolk plasma dry matter and 2 to 5 % glycerol.

5. Use as claimed in any one of claims 1 to 4, **characterised in that** the base medium contains additives such as antibiotics or antifungals.

## Patentansprüche

1. Verwendung eines Sperma-Verdünnungsmediums, gebildet aus:
- einem Basismedium, umfassend die Inhaltsstoffe geeignet zur Sperma-Verdünnung einer bestimmten Art, das eine Lösung von Mineralsalzen und Kohlenhydraten beinhaltet, und keine Milch beinhaltet;
- zwischen 1 und 100 g/l natives Phosphocaseinat;
- von 0,5 bis 12,5 Gew.-% Eigelb-Trockensubstanz oder von 0,4 bis 10 Gew.-% Eigelbplasma-Trockensubstanz; und
- 1 bis 10% Glycerin;
um die Befruchtungstauglichkeit des Spermiums, geschwächt durch das Einfrieren oder durch das Sortieren der Spermatozoiden mittels Durchflusszytometrie, zu verbessern.

2. Verwendung gemäß Anspruch 1, **dadurch gekennzeichnet, dass** das Sperma-Verdünnungsmedium zwischen 10 und 50 g/l natives Phosphocaseinat umfasst.

3. Verwendung gemäß Anspruch 1 oder 2, **dadurch gekennzeichnet, dass** das Sperma-Verdünnungsmedium 0, 5 bis 5 Gew.-% Eigelb-Trockensubstanz oder 0,4 bis 4 Gew.-% Eigelbplasma-Trockensubstanz umfasst.

4. Verwendung gemäß Anspruch 3, **dadurch gekennzeichnet, dass** das Sperma-Verdünnungsmedium 1 bis 2,5 Gew.-% Eigelb oder 0,8 bis 2 Gew.-% Eigelbplasma-Trockensubstanz und 2 bis 5% Glycerin umfasst.

5. Verwendung gemäß einem der Ansprüche 1 bis 4, **dadurch gekennzeichnet , dass** das Basismedium Zusätze umfasst, wie zum Beispiel Antibiotika oder Anti-Pilzmittel.
